# EUROPÄISCHE PATENTSCHRIFT

(11) **EP 3 090 709 B1**
(45) Veröffentlichungstag und Bekanntmachung des Hinweises auf die Patenterteilung: **01.05.2019**
(21) Anmeldenummer: 16168365.1
(22) Anmeldetag: 04.05.2016
(51) Int. Cl.: A61F 5/01

(54) **ORTHESE ZUR THERAPEUTISCHEN BEHANDLUNG**
ORTHOSIS FOR TREATMENT
ORTHESE DE TRAITEMENT THERAPEUTIQUE

(30) Priorität: 08.05.2015 DE 202015003437 U
(43) Veröffentlichungstag der Anmeldung: 09.11.2016
(73) Patentinhaber: Thuasne Deutschland GmbH, 30938 Burgwedel (DE)
(72) Erfinder: Schöbel, Herbert, 30938 Burgwedel (DE); Schneider-Nieskens, Reinhold, 29223 Celle (DE)
(74) Vertreter: Siekmann, Gunnar

(56) Entgegenhaltungen:
- EP-A1- 2 590 603
- DE-A1- 19 811 925
- DE-T5-112005 000 570
- US-A1- 2008 300 524
- US-A1- 2013 245 523

## Beschreibung

Die Erfindung betrifft eine Orthese zur therapeutischen Behandlung, insbesondere zur Behandlung von patello-femoralen Schmerzen, aufweisend einen Grundkörper zur Anlage um ein Körperteil, ein Wirkelement, mit dem bei angelegter Orthese Druck auf eine vorbestimmte Körperregion ausübbar ist, so dass sich diese nur in eine vorbestimmte Richtung bewegen kann, und eine Verstelleinrichtung, die dem Wirkelement derart zugeordnet ist, dass durch Betätigen der Verstelleinrichtung der Druck auf die Körperregion einstellbar ist, wobei die Verstelleinrichtung wenigstens ein Zugelement aufweist, das Zugelement mit einem ersten Ende an dem Wirkelement befestigt ist und die Verstelleinrichtung wenigstens ein Befestigungsmittel aufweist, mit dem ein zweites Ende des Zugelements an dem Grundkörper festsetzbar ist, wobei das Zugelement einen Verstellweg mit wenigstens einer Zugelementumlenkung aufweist.

Der mit dem Wirkelement auf die Körperregion ausgeübte Druck bewirkt bei einer Orthese zur Behandlung von patello-femoralen Schmerzen eine Beeinflussung des Patellagleitweges, wobei die Kniescheibe medialisiert wird, das heißt aus einer außermittigen Lage in eine mittige Lage zum Kniegelenk gezogen wird. Diese Korrektur der Kniescheibe reduziert Schmerzen und verhindert teilweise auch eine Luxation der Kniescheibe.

Um eine Orthese an einen Patienten anpassen und für diesen einstellen zu können, weisen entsprechende Orthesen zumeist eine Verstelleinrichtung auf. Bei Orthesen aus dem Stand der Technik erfolgt die Einstellung des Wirkelements zur Kniescheibe dabei zumeist direkt linear, wobei sich Wirkelement und Verstelleinrichtung nur bedingt an eine durch das jeweilige Kniegelenk vorgegebene Körperkontur anpassen lassen. Eine solche Orthese geht beispielsweise aus der DE 11 2005 000 570 T5 hervor, bei der mehrere Zugelemente an Verbindungspunkten an das Wirkelement angreifen können und an einem Grundkörper befestigt werden.

Ein anderes Zugelement einer Orthese geht aus der DE 198 11 925 A1 hervor, wobei zwei Fixierschienen über einen Bewegungsmechanismus miteinander verbunden sind. Der Bewegungsmechanismus soll dabei ein Zugelement umfassen, das seitlich zu dem zu stabilisierenden Gelenk geführt ist. Dieses Zugelement ist als Seilring ausgebildet und außenseitig an den Fixierschienen befestigt. Gemäß einer vorteilhaften Ausgestaltung ist dieses als Seilring ausgebildete Zugelement über Umlenklager an den Seitenflächen der zweiten Fixierschiene umlenkbar und verläuft von dort zur Vorderseite der zweiten Fixierschiene. An der Vorderseite soll ein Verstellelement für das Zugmittel angeordnet sein.

Aufgabe der Erfindung ist es daher eine Orthese bereitzustellen, die sich in verbesserter Weise an eine jeweilige Körperkontur anpasst und eine zielgerichtete Beeinflussung der vorbestimmten Körperregion ermöglicht und eine sichere und einfachere Handhabung gewährleistet.

Die Lösung dieser Aufgabe erfolgt mit einer Orthese mit den Merkmalen des Patentanspruchs 1. Weiterbildungen und vorteilhafte Ausgestaltungen der Erfindung sind in den nachgeordneten Patentansprüchen 2 bis 15 angegeben.

Die Orthese zur therapeutischen Behandlung, insbesondere zur Behandlung von patello-femoralen Schmerzen, aufweisend einen Grundkörper zur Anlage um ein Körperteil, ein Wirkelement, mit dem bei angelegter Orthese Druck auf eine vorbestimmte Körperregion ausübbar ist, so dass sich diese nur in eine vorbestimmte Richtung bewegen kann, und eine Verstelleinrichtung, die dem Wirkelement derart zugeordnet ist, dass durch Betätigen der Verstelleinrichtung der Druck auf die Körperregion einstellbar ist, wobei die Verstelleinrichtung wenigstens ein Zugelement aufweist, das Zugelement mit einem ersten Ende an dem Wirkelement befestigt ist und die Verstelleinrichtung wenigstens ein Befestigungsmittel aufweist, mit dem ein zweites Ende des Zugelements an dem Grundkörper festsetzbar ist, , wobei das Zugelement einen Verstellweg mit wenigstens einer Zugelementumlenkung aufweist, zeichnet sich erfindungsgemäß dadurch aus, dass das Zugelement durch wenigstens eine Ausnehmung des Grundkörpers an eine Außenseite dieses geführt ist. Mit der Zugelementumlenkung ist auf einfache Weise eine ergonomische Anpassung an eine räumliche Kontur eines Körperteils ermöglicht, so dass eine optimale Position des Wirkelements an der Körperregion sichergestellt ist. Zudem ist erreicht, dass das Wirkelement erfindungsgemäß mit einem einzigen Zugelement eingestellt und an einen Patienten angepasst werden kann, so dass auch eine einfachere Handhabung gewährleistet ist.

Gemäß einer ersten Ausgestaltung weist das Befestigungsmittel wenigstens einen Klettverschluss auf, mit dem das Wirkelement stufenlos zum Grundkörper verstellbar ist und so eine individuelle Anpassung des Druckes auf die Körperregion des Patienten ermöglicht. Dabei umfasst der Klettverschluss vorteilhafterweise wenigstens einen Klettstreifen, insbesondere einen Klettvelourstreifen. Die Länge des Klettstreifens ausgehend von der Zugelementumlenkung bestimmt dabei die maximale Länge des Verstellweges innerhalb dem das Wirkelement zu dem Grundkörper festlegbar ist.

Ergänzend oder alternativ zu einem Klettverschluss kann das Befestigungsmittel gemäß einer zweiten oder einer dritten Ausgestaltung einen Einrastverschluss aufweisen, mit dem das zweite Ende des Zugelementes an dem Grundkörper festsetzbar ist. Ein entsprechendes Einrastelement stellt eine besonders sichere Verbindung zwischen Zugelement und Grundkörper der Orthese dar und gewährleistet zudem, dass das Wirkelement bei jedem Anlegen der Orthese nach Spannen des Zugelements den gleichen Druck auf die Körperregion ausübt, indem das Zugelement einfach wieder an gleicher Stelle eingerastet wird.

Bei Kombination von Klettverschluss und Einrastverschluss stellt der Klettverschluss eine ideale Möglichkeit dar, die Orthese bei einer ersten Anprobe auf optimale Weise, zum Beispiel durch Fachpersonal, an einen Patienten anzupassen. Der Klettverschluss braucht dann im Verlauf der weiteren Behandlung nicht mehr gelöst zu werden, sondern es kann auf einfache Weise nur der Einrastverschluss gelöst werden, um die Körperregion immer mit dem gleichen Druck durch das Wirkelement zu beaufschlagen, ohne dass die alte Position des Wirkelements erst wiedergefunden werden muss.

Das Zugelement selbst weist nach einer Weiterbildung wenigstens ein Zugseil auf. Dieses bildet dann die Verbindung zwischen Wirkelement und Befestigungsmittel der Verstelleinrichtung aus.

Um eine einfache Handhabung der Verstelleinrichtung zu gewährleisten, ist vorgesehen, dass das Zugelement durch wenigstens eine Ausnehmung, insbesondere eine schlitzförmige Ausnehmung, des Grundkörpers an eine Außenseite dieses geführt ist. Die Verstellung des innenliegenden, das heißt an einer Innenseite des Grundkörpers der Orthese oder zwischen zwei Lagen des Grundkörpers der Orthese angeordneten, Wirkelements kann so auf einfache Weise außen an dem Grundkörper erfolgen. Die Anzahl der schlitzförmigen Ausnehmungen ist jeweils abhängig von der Anzahl der Zugseile, wobei zumeist je Zugseil eine schlitzförmige Ausnehmung vorgesehen ist. Damit das Zugseil im Bereich des Schlitzes sicher geführt ist, kann weiter vorgesehen sein, dass wenigstens ein Zugseil, insbesondere alle Zugseile, über eine bandartige Lasche, insbesondere eine bandartige elastische oder teilelastische Lasche, mit dem Wirkelement verbunden ist. Die bandartige Lasche erstreckt sich dabei bis in den Bereich des Zugelements, der durch die schlitzförmige Ausnehmung nach außen geführt ist. Um Bewegungen quer zur vorbestimmten Zugrichtung des Zugelements im Bereich der Ausnehmung zu minimieren und eine verbesserte Führung des Zugelements zu erreichen, weist die bandartige Lasche bevorzugt eine Breite auf, die gleich oder geringfügig kleiner ist als die Breite der jeweiligen schlitzförmigen Ausnehmung.

In bevorzugter Ausgestaltung stellt die Orthese eine Knieorthese zur Behandlung von patello-femoralen Schmerzen dar, wobei das Körperteil, um das der Grundkörper anlegbar ist, ein Kniegelenk ist und wobei mit dem Wirkelement, bei angelegter Orthese, Druck auf die Kniescheibe ausübbar ist. Vorteilhafterweise ist das zweite Ende des Zugelements mit dem Befestigungsmittel ausgehend vom Kniegelenk dann in proximale Richtung an dem Grundkörper festsetzbar, das heißt in Richtung Rumpf eines Patienten der mit der Orthese behandelt werden soll. Beim Anlegen der Orthese kann der Patient das Wirkelement dann durch Heranziehen des Zugelements zum Körper hin so fixieren, dass die Kniescheibe medialisiert ist. Die Orthese weist dabei zumindest in proximaler Richtung eine ausreichende Stabilität auf, durch die sichergestellt ist, dass sich die Orthese nach Festlegen des Zugelements an dem Grundkörper nicht in Längserstreckung, das heißt in proximaler oder distaler Richtung, zusammenzieht. Sämtliche auf das Wirkelement beaufschlagte Zugkraft wirkt somit in mediale Richtung, wobei eine Veränderung des Verstellweges ausschließlich zu einer Veränderung des Abstands von Wirkelement und Zugelementumlenkung zueinander führt und der Beeinflussung des Patellagleitweges dient. Besonders leicht mit einer Hand zu handhaben ist das Befestigungsmittel, wenn das zweite Ende zudem medial zum Kniegelenk an dem Grundkörper der Orthese festsetzbar ist.

Besonders vorteilhaft gestaltet es sich dabei, wenn das Zugelement ein erstes Zugseil und ein zweites Zugseil aufweist, wobei das erste Zugseil in einem proximalen Bereich des Wirkelementes an dieses angreift und das zweite Zugseil in einem distalen Bereich des Wirkelementes an dieses angreift und mit den Zugseilen bei angelegter Orthese durch das Wirkelement Druck in medialer Richtung auf die Kniescheibe des Kniegelenkes ausübbar ist. Zwei oder mehr Zugseile haben dabei den Vorteil, dass die mit dem Zugelement auf das Wirkelement aufgebrachten Kräfte gleichmäßiger auf einer größeren Fläche verteilt werden und einzelne Bereiche des Wirkelements nicht übermäßig beansprucht werden.

Als Wirkelement ist nach einer Weiterbildung eine C-förmige Pelotte vorgesehen, die innenseitig an dem Grundkörper oder zwischen zwei Lagen des Grundkörpers angeordnet ist. Die C-förmige Pelotte umschließt die Kniescheibe dabei vorteilhafterweise zu drei Seiten und gewährleistet so, dass der Patellagleitweg gezielt in eine Richtung beeinflusst beziehungsweise gelenkt wird, um diese zu medialisieren.

Das Zugelement aus erstem und zweitem Zugseil ist dann bevorzugt an einander entgegengesetzten Enden des als C-förmige Pelotte ausgebildeten Wirkelements befestigt. Beide Zugseile sind dann der offenen Seite des C-förmigen Wirkelements zugeordnet beziehungsweise an den die Öffnung begrenzenden Enden angeordnet.
Die Zugelementumlenkung ist in weiterer Ausgestaltung durch wenigstens eine Schlaufe ausgebildet, durch die das Zugelement hindurchgeführt ist. Diese Schlaufe ist so angeordnet, dass der Verstellweg des Zugelements bei gestrecktem Bein im Wesentlichen um einen Winkel von 90°, insbesondere einem Winkel zwischen 85° und 95°, verschwenkt wird, so dass das vom Wirkelement lateral abgehende Zugelement in proximale Richtung umgelenkt wird. Damit mehrere Zugseile jeweils parallel zueinander führbar und umlenkbar sind, ist wiederum je Zugseil mindestens eine Schlaufe vorzusehen. Insbesondere den weiter in distaler Richtung angeordneten Zugseilen können auch mehrere Schlaufen zugeordnet sein, um eine sichere Führung der Zugseile im Bereich der Zugelementumlenkung sicherzustellen. Diese Schlaufen sind nach einer Weiterbildung außenseitig an dem Grundkörper angeordnet, das heißt an diesem befestigt.

Zum Schutz vor äußeren Einflüssen, zum Beispiel, um zu vermeiden, dass das Zugelement, die Zugseile oder die Schlaufen an etwas hängenbleiben und möglicherweise abreißen kann die Zugelementumlenkung zumindest teilweise unterhalb einer Abdeckung angeordnet sein. Diese Abdeckung kann in einem Bereich einer in den Grundkörper eingearbeiteten Gelenkschiene angeordnet sein, in dem auch die Zugelementumlenkung angeordnet ist. Die Gelenkschiene bildet dabei einen stabileren Bereich der Orthese aus. Die auf das Wirkelement beaufschlagte Zugkraft führt so dazu, dass sich die Orthese nach Befestigen des Zugelements lediglich in lateraler Richtung zusammenzieht, in Längsrichtung eines Beines mit einem zu behandelnden Kniegelenk dagegen eine gleichbleibende, unveränderte Länge aufweist. Alternativ kann die Zugelementumlenkung auch in einem Bereich eines an dem Grundkörper vorgesehenen Gelenkscharniers angeordnet sein, wobei dieses Gelenkscharnier zumeist zwei Teile einer Gelenkschiene miteinander verbindet.

Der Grundkörper selbst ist in bevorzugter Ausgestaltung mantelförmig oder schlauchförmig ausgebildet und wird beim Anlegen der Orthese vom distalen Ende des Beines bis zum Kniegelenk gezogen. Um den Grundkörper der Orthese anlegen zu können muss dieser daher elastische Eigenschaften aufweisen, die in verschiedenen Bereichen des Grundkörpers unterschiedliche Dehneigenschaften aufweisen können. Ein solcher Grundkörper kann auf einfache Weise aus einem Textil, insbesondere aus einem gestrickten, gewirkten oder gewebten Textil oder einer Kombination der unterschiedlichen Textilien bestehen, das je nach Gewebe- oder Textilart unterschiedliche elastische Eigenschaften aufweist und somit einerseits ausreichend dehnbar ist um die Orthese über Fuß und Unterschenkel zu ziehen und andererseits am Kniegelenk eine optimale Passform zu gewährleisten.

Ein Ausführungsbeispiel der Erfindung geht aus der Zeichnung hervor. Es zeigen:
- Figur 1:: eine perspektivische Darstellung einer angelegten Orthese;
- Figur 2:: eine schematische Darstellung einer Verstelleinrichtung mit einem Wirkelement der erfindungsgemäßen Orthese gemäß Figur 1; und
- Figur 3:: eine Detaildarstellung der Verstelleinrichtung gemäß Figur 2.

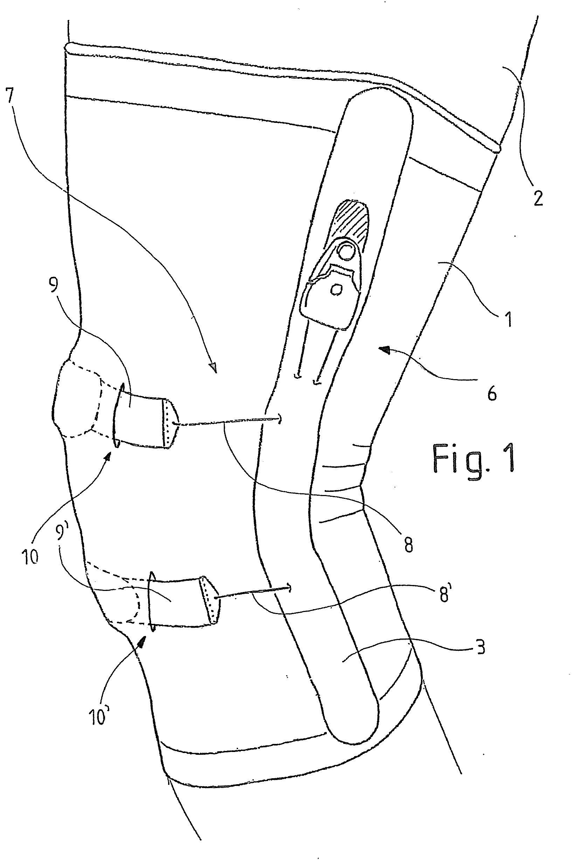

In Figur 1 ist eine Orthese zur Behandlung von patello-femoralen Schmerzen dargestellt, die einen Grundkörper 1 zur Anlage um ein zu behandelndes Kniegelenk eines Beines 2 aufweist. Dieser Grundkörper 1 ist mantel- bzw. schlauchförmig ausgebildet und besteht aus einem elastischen Textil, wodurch der Grundkörper 1 mit seiner Innenseite eng an Kniegelenk und an Bein 2 anliegt. An dem Grundkörper 1 ist eine lateral angeordnete Gelenkschiene 3 ausgebildet, die sich von einem distalen zu einem proximalen Ende des Grundkörpers 1 erstreckt und das Kniegelenk stützt. Außenseitig weist diese Gelenkschiene 3 einen Klettstreifen 4, insbesondere einen Klettvelourstreifen, auf, an dem ein Einrastverschluss 5 befestigt ist. Der zweiteilige Einrastverschluss 5 bildet dabei zusammen mit dem Klettstreifen 4 ein Befestigungsmittel eines Zugelements 6 einer Verstelleinrichtung 7, mit der ein Wirkelement der Orthese an einen Patienten angepasst werden kann. Das Zugelement 6 der Verstelleinrichtung 7 umfasst weiter zwei Zugseile 8, 8', die mit einem ersten Ende jeweils über eine bandartige Lasche 9, 9' mit dem Wirkelement verbunden sind und an dessen zweiten Enden der Einrastverschluss 5 angeordnet ist, mit dem das Zugelement 6 an dem Klettstreifen 4 befestigt ist.

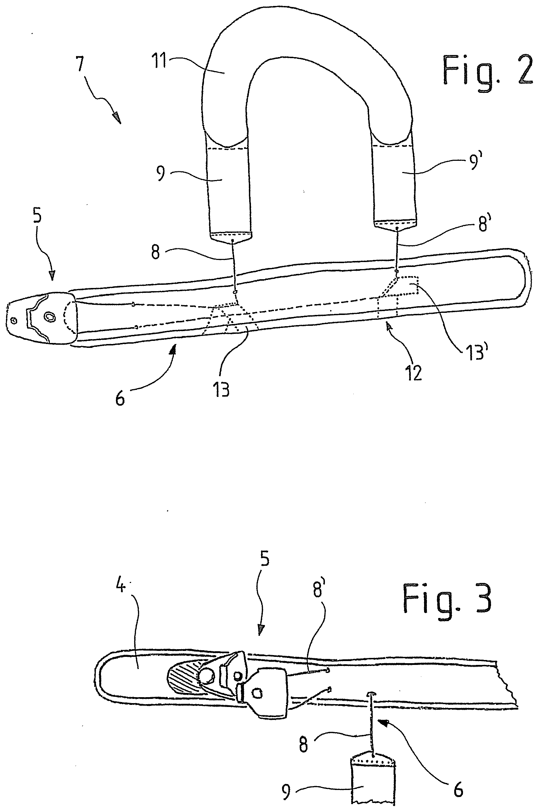

Die beiden bandartigen Laschen 9, 9' sind jeweils durch eine schlitzförmige Ausnehmung 10, 10' in dem Grundkörper 1 zu dessen an dem Bein 2 anliegenden Innenseite geführt, an der das in Figur 2 dargestellte Wirkelement 11 an dem Grundkörper 1 befestigt ist. Aus Figur 2 geht weiter hervor, dass als Wirkelement 11 eine C-förmige Pelotte vorgesehen ist, an deren beiden Enden des C-förmigen Bogens jeweils eine der bandartigen Laschen 9, 9' des Zugelements 6 angreift, wobei ein Ende der C-förmigen Pelotte distal und ein Ende proximal angeordnet ist. Die bandartige Lasche 9 mit dem Zugseil 8 ist dem proximalen Ende der C-förmigen Pelotte und die bandartige Lasche 9' mit dem Zugseil 8' dem distalen Ende der C-förmigen Pelotte zugeordnet, wobei beide Zugseile 8, 8' in lateraler Richtung von der C-förmigen Pelotte weggeführt sind. Unterhalb des zumindest abschnittsweise eine Abdeckung für eine Zugelementumlenkung 12 bildenden Klettstreifens 4 sind zwei die Zugelementumlenkung 12 ausbildende Schlaufen 13, 13' vorgesehen. Alle unterhalb der aus dem Klettstreifen 4 gebildeten Abdeckung angeordneten Bereiche sind dabei gepunktet beziehungsweise gestrichelt dargestellt, um den Verlauf der Zugseile 8, 8' auch unterhalb der Abdeckung zu verdeutlichen. Durch die Zugelementumlenkung 12 ist erreicht, dass der Patient das Wirkelement 11 durch einfaches Heranziehen des Einrastverschlusses 5 in proximale Richtung einstellen und damit den Patellagleitweg in gewünschter Weise beeinflussen kann.

Um die Einstellung des Druckes auf die Kniescheibe nicht bei jedem Anlegen der Orthese von neuem ermitteln und festlegen zu müssen, kann auch nur ein unmittelbar mit den Zugseilen 8, 8' verbundener erster Teil des Einrastverschlusses 5 gelöst werden, wie in Figur 3 dargestellt. Ein zweiter Teil des Einrastverschlusses 5 verbleibt dann an dem Klettstreifen 4, so dass ein einmal eingestellter Druck auf die Kniescheibe, der auf den jeweiligen Patienten abgestimmt ist auch nach einem Ab- und Anlegen der Orthese nicht neu eingestellt werden muss.

Alle in der vorstehenden Beschreibung und in den Ansprüchen genannten Merkmale sind in einer beliebigen Auswahl mit den Merkmalen des unabhängigen Anspruchs kombinierbar. Die Offenbarung der Erfindung ist somit nicht auf die beschriebenen beziehungsweise beanspruchten Merkmalskombinationen beschränkt, vielmehr sind alle im Rahmen der Erfindung sinnvollen Merkmalskombinationen als offenbart zu betrachten.

## Patentansprüche

1. Orthese zur therapeutischen Behandlung, insbesondere zur Behandlung von patello-femoralen Schmerzen, aufweisend einen Grundkörper (1) zur Anlage um ein Körperteil, ein Wirkelement (11), mit dem bei angelegter Orthese Druck auf eine vorbestimmte Körperregion ausübbar ist, so dass sich diese nur in eine vorbestimmte Richtung bewegen kann, und eine Verstelleinrichtung (7), die dem Wirkelement (11) derart zugeordnet ist, dass durch Betätigen der Verstelleinrichtung (7) der Druck auf die Körperregion einstellbar ist, wobei die Verstelleinrichtung (7) wenigstens ein Zugelement (6) aufweist, das Zugelement (6) mit einem ersten Ende an dem Wirkelement (11) befestigt ist und die Verstelleinrichtung (7) wenigstens ein Befestigungsmittel aufweist, mit dem ein zweites Ende des Zugelements (6) an dem Grundkörper (1) festsetzbar ist, wobei das Zugelement (6) einen Verstellweg mit wenigstens einer Zugelementumlenkung (12) aufweist,
**dadurch gekennzeichnet,**
**dass** das Wirkelement (11) an einer Innenseite des Grundkörpers (1) der Orthese oder zwischen zwei Lagen des Grundkörpers (1) der Orthese angeordnet ist, und das Zugelement (6) durch wenigstens eine Ausnehmung des Grundkörpers (1) an eine Außenseite des Grundkörpers geführt ist.

2. Orthese nach Anspruch 1, **dadurch gekennzeichnet, dass** das Befestigungsmittel wenigstens einen Klettverschluss umfasst.

3. Orthese nach einem der Ansprüche 1 oder 2, **dadurch gekennzeichnet, dass** das Befestigungsmittel einen Einrastverschluss (5) aufweist, mit dem das zweite Ende des Zugelementes (6) an dem Grundkörper (1) festsetzbar ist.

4. Orthese nach einem der Ansprüche 1 bis 3, **dadurch gekennzeichnet, dass** das Zugelement (6) wenigstens ein Zugseil (8, 8') aufweist.

5. Orthese nach einem der Ansprüche 1 bis 4, **dadurch gekennzeichnet, dass** wenigstens ein Zugseil (8, 8'), insbesondere alle Zugseile (8, 8'), über eine bandartige Lasche (9, 9') mit dem Wirkelement (11) verbunden ist.

6. Orthese nach einem der Ansprüche 1 bis 5, **dadurch gekennzeichnet, dass** die Orthese zur Behandlung von patello-femoralen Schmerzen an einem Kniegelenk vorgesehen ist.

7. Orthese nach Anspruch 6, **dadurch gekennzeichnet, dass** das zweite Ende des Zugelements (6) mit dem Befestigungsmittel ausgehend vom Kniegelenk in proximale Richtung an dem Grundkörper (1) festsetzbar ist.

8. Orthese nach einem der Ansprüche 6 oder 7, **dadurch gekennzeichnet, dass** das zweite Ende medial zum Kniegelenk an dem Grundkörper (1) festsetzbar ist.

9. Orthese nach einem der Ansprüche 1 bis 8, **dadurch gekennzeichnet, dass** die Orthese eine Knieorthese zur Behandlung von patello-femoralen Schmerzen darstellt, wobei das Körperteil, um das der Grundkörper anlegbar ist, ein Kniegelenk ist und wobei mit dem Wirkelement, bei angelegter Orthese, Druck auf die Kniescheibe ausübbar ist.

10. Orthese nach einem der Ansprüche 6 bis 9, **dadurch gekennzeichnet, dass** das Zugelement (6) wenigstens ein erstes Zugseil (8) und ein zweites Zugseil (8') aufweist, wobei das erste Zugseil (8) in einem proximalen Bereich an das Wirkelement (11) angreift und das zweite Zugseil (8') in einem distalen Bereich an das Wirkelement (11) angreift und mit den Zugseilen (8, 8') bei angelegter Orthese durch das Wirkelement (11) Druck in medialer Richtung auf die Kniescheibe des Kniegelenks ausübbar ist.

11. Orthese nach einem der Ansprüche 1 bis 10, **dadurch gekennzeichnet, dass** das Wirkelement (11) eine C-förmige Pelotte ist.

12. Orthese nach einem der Ansprüche 1 bis 11, **dadurch gekennzeichnet, dass** die Zugelementumlenkung (12) durch wenigstens eine Schlaufe (13, 13') ausgebildet ist, durch die das Zugelement (6) hindurchgeführt ist.

13. Orthese nach Anspruch 12, **dadurch gekennzeichnet, dass** jedem Zugseil (8, 8') des Zugelements (6) wenigstens eine Schlaufe (13, 13') zugeordnet ist.

14. Orthese nach einem der Ansprüche 1 bis 13, **dadurch gekennzeichnet, dass** die Zugelementumlenkung (12) zumindest teilweise unterhalb einer Abdeckung angeordnet ist.

15. Orthese nach einem der Ansprüche 1 bis 14, **dadurch gekennzeichnet, dass** der Grundkörper (1) mantelförmig ausgebildet ist.

## Claims

1. An orthotic for therapeutic treatment, in particular for the treatment of patellofemoral pain, comprising a main body (1) for placing around a body part, an operating element (11) with which pressure is exerted on a designated region of the body when the orthotic is in position, so that the region of the body can only move in a designated direction, and an adjustment device (7) which is associated with the operating element (11) in a manner such that the pressure on the region of the body can be adjusted by actuating the adjustment device (7), wherein the adjustment device (7) comprises at least one traction element (6), a first end of the traction element (6) is secured to the operating element (11) and the adjustment device (7) comprises at least one fastening means with which a second end of the traction element (6) can be fixed to the main body (1), wherein the traction element (6) comprises an adjustment way with at least one guide (12) for the traction element,
**characterized in**
**that** the operating element (11) is disposed on an inner surface of the main body (1) of the orthotic or between two plies of the main body (1) of the orthotic, and
the traction element (6) is guided through at least one recess of the main body (1) onto an outer surface of the main body.

2. The orthotic as claimed in claim 1, **characterized in that** the fastening means comprises at least one hook and loop fastening.

3. The orthotic as claimed in claim 1 or claim 2, **characterized in that** the fastening means comprises a clip fastener (5) with which the second end of the traction element (6) can be fixed to the main body (1) .

4. The orthotic as claimed in one of claims 1 to 3, **characterized in that** the traction element (6) comprises at least one traction cord (8, 8').

5. The orthotic as claimed in one of claims 1 to 4, **characterized in that** at least one traction cord (8, 8'), in particular all of the traction cords (8, 8'), is connected to the operating element (11) via a strap-like tab (9, 9').

6. The orthotic as claimed in one of claims 1 to 5, **characterized in that** the orthotic is provided for the treatment of patellofemoral pain in a knee joint.

7. The orthotic as claimed in claim 6, **characterized in that** the second end of the traction element (6) with the fastening means can be fixed to the main body (1) in the proximal direction with respect to the knee joint.

8. The orthotic as claimed in claim 6 or claim 7, **characterized in that** the second end can be fixed to the main body (1) medially with respect to the knee joint.

9. The orthotic as claimed in one of claims 1 to 8, **characterized in that** the orthotic constitutes a knee orthotic for the treatment of patellofemoral pain, wherein the body part about which the main body can be placed is a knee joint and wherein pressure can be exerted on the patella with the operating element when the orthotic is in position.

10. The orthotic as claimed in one of claims 6 to 9, **characterized in that** the traction element (6) comprises at least one first traction cord (8) and one second traction cord (8'), wherein the first traction cord (8) engages with the operating element (11) in a proximal region and the second traction cord (8') engages with the operating element (11) in a distal region and when the orthotic is in position, pressure can be exerted on the patella of the knee joint in the medial direction with the traction cords (8, 8'), by means of the operating element (11).

11. The orthotic as claimed in one of claims 1 to 10, **characterized in that** the operating element (11) is a C-shaped pelotte.

12. The orthotic as claimed in one of claims 1 to 11, **characterized in that** the traction element guide (12) is formed by at least one loop (13, 13') through which the traction element (6) is guided.

13. The orthotic as claimed in claim 12, **characterized in that** each traction cord (8, 8') of the traction element (6) is associated with at least one loop (13, 13').

14. The orthotic as claimed in one of claims 1 to 13, **characterized in that** at least a portion of the traction element guide (12) is disposed beneath a cover.

15. The orthotic as claimed in one of claims 1 to 14, **characterized in that** the main body (1) is configured in the shape of a sheath.

## Revendications

1. Orthèse, destinée au traitement thérapeutique, notamment au traitement de douleurs patellofemorales, comportant un corps de base (1), destiné à être appliqué sur une partie du corps, un élément actif (11), à l'aide duquel, lorsque l'orthèse est appliquée, une pression est susceptible d'être exercée sur une région prédéfinie du corps, de sorte que celle-ci ne puisse se déplacer que dans une direction prédéfinie et un système d'ajustage (7) qui est associé à l'élément actif (11), de telle sorte que par actionnement du système d'ajustage (7), la pression sur la région du corps soit réglable, le système d'ajustage (7) comportant au moins un élément de traction (6), l'élément de traction (6) étant fixé par une première extrémité sur l'élément actif (11) et le système d'ajustage (7) comportant au moins un moyen de fixation à l'aide duquel une deuxième extrémité de l'élément de traction (6) est susceptible d'être immobilisée sur le corps de base (1), l'élément de traction (6) comportant une course d'ajustage pourvue d'au moins un renvoi de l'élément de traction (12),
**caractérisée en ce**
**que** l'élément actif (11) est placé sur une face intérieure du corps de base (1) de l'orthèse ou entre deux couches du corps de base (1) de l'orthèse et
**en ce que** l'élément de traction (6) est guidé par au moins un évidement du corps de base (1) sur une face extérieure du corps de base.

2. Orthèse selon la revendication 1, **caractérisée en ce que** le moyen de fixation comprend au moins une fermeture autoagrippante.

3. Orthèse selon l'une quelconque des revendications 1 ou 2, **caractérisée en ce que** le moyen de fixation comporte une fermeture par enclenchement (5), à l'aide de laquelle la deuxième extrémité de l'élément de traction (6) est susceptible d'être immobilisée sur le corps de base (1).

4. Orthèse selon l'une quelconque des revendications 1 à 3, **caractérisée en ce que** l'élément de traction (6) comporte au moins un câble de traction (8, 8').

5. Orthèse selon l'une quelconque des revendications 1 à 4, **caractérisée en ce qu'**au moins un câble de traction (8, 8'), notamment tous les câbles de traction (8, 8'), est assemblés par l'intermédiaire d'une patte (9, 9') en forme de bande avec l'élément actif (11).

6. Orthèse selon l'une quelconque des revendications 1 à 5, **caractérisée en ce que** l'orthèse est prévue pour le traitement de douleurs patellofemorales sur une articulation du genou.

7. Orthèse selon la revendication 6, **caractérisée en ce que** la deuxième extrémité de l'élément de traction (6) est susceptible d'être immobilisée à l'aide du moyen de fixation, à partir de l'articulation du genou, dans la direction proximale sur le corps de base (1).

8. Orthèse selon l'une quelconque des revendications 6 ou 7, **caractérisée en ce que** la deuxième extrémité est susceptible d'être immobilisée en direction médiale par rapport à l'articulation du genou sur le corps de base (1).

9. Orthèse selon l'une quelconque des revendications 1 à 8, **caractérisée en ce que** l'orthèse est une orthèse du genou, destinée au traitement de douleurs patellofemorales, la partie du corps sur laquelle le corps de base est applicable étant une articulation de genou et à l'aide de l'élément actif, lorsque l'orthèse est appliquée, une pression étant susceptible d'être exercée sur la rotule.

10. Orthèse selon l'une quelconque des revendications 6 à 9, **caractérisée en ce que** l'élément de traction (6) comporte au moins un premier câble de traction (8) et un deuxième câble de traction (8'), le premier câble de traction (8) s'engageant dans une zone proximale sur l'élément actif (11) et le deuxième câble de traction (8') s'engageant dans une zone distale sur l'élément actif (11) et avec les câbles de traction (8, 8'), lorsque l'orthèse est appliquée, une pression étant applicable par l'élément actif (11) en direction médiale sur la rotule de l'articulation de genou.

11. Orthèse selon l'une quelconque des revendications 1 à 10, **caractérisée en ce que** l'élément actif (11) est une pelote en forme de C.

12. Orthèse selon l'une quelconque des revendications 1 à 11, **caractérisée en ce que** le renvoi de l'élément de traction (12) est conçu par au moins une boucle (13, 13') à travers laquelle l'élément de traction (6) est guidé.

13. Orthèse selon la revendication 12, **caractérisée en ce qu'**à chaque câble de traction (8, 8') de l'élément de traction (6) est associée au moins une boucle (13, 13').

14. Orthèse selon l'une quelconque des revendications 1 à 13, **caractérisée en ce que** le renvoi de l'élément de traction (12) est placé au moins partiellement sous un cache.

15. Orthèse selon l'une quelconque des revendications 1 à 14, **caractérisée en ce que** le corps de base (1) est conçu en forme d'enveloppe.
